# EUROPEAN PATENT SPECIFICATION

(11) **EP 1 954 295 B1**
(45) Date of publication and mention of the grant of the patent: **06.08.2014**
(21) Application number: 06843850.6
(22) Date of filing: 18.11.2006
(51) Int. Cl.: A61K 31/7072, A61P 27/02

(54) **COMPOSITION HAVING EFFECT ON TREATMENT AND PREVENTION OF DRY EYE SYNDROME**
ZUSAMMENSETZUNG MIT AUSWIRKUNG AUF DIE BEHANDLUNG UND PRÄVENTION DES TROCKENEN AUGES
COMPOSITION AYANT UN EFFET SUR LE TRAITEMENT ET LA PREVENTION DE LA KERATOCONJONCTIVITE SECHE

(30) Priority: 28.11.2005 KR 20050113942
(43) Date of publication of application: 13.08.2008
(73) Proprietor: Md Bioalpha Co., Ltd., 302-120 Daejeon (KR)
(72) Inventor: PARK, Myung-Gyu, Gyeonggi-do 446-908 (KR); KUON, Min-Kyung, Gyeonggi-do 441-350 (KR)
(74) Representative: Stona, Daniel
(86) International application number: PCT/KR2006/004866
(87) International publication number: WO 2007/061200

(56) References cited:
- WO-A1-98/34593
- WO-A2-00/50024
- KR-A- 20030 022 740
- KR-A- 20050 111 670
- US-A1- 2003 008 834
- US-B1- 6 713 458
- KEPPLER D O ET AL: "Selective uridine triphosphate deficiency induced by D-galactosamine in liver and reversed by pyrimidine nucleotide precursors. Effect on ribonucleic acid synthesis.", THE JOURNAL OF BIOLOGICAL CHEMISTRY 10 JAN 1974, vol. 249, no. 1, 10 January 1974 (1974-01-10), pages 211-216, XP002698949, ISSN: 0021-9258
- CONNOLLY G P ET AL: "Uridine and its nucleotides: biological actions, therapeutic potentials", TRENDS IN PHARMACOLOGICAL SCIENCES, ELSEVIER, HAYWARTH, GB, vol. 20, no. 5, May 1999 (1999-05), pages 218-225, XP004170246, ISSN: 0165-6147, DOI: 10.1016/S0165-6147(99)01298-5
- JUMBLATT J E ET AL: "Regulation of ocular mucin secretion by P2Y2 nucleotide receptors in rabbit and human conjunctiva.", EXPERIMENTAL EYE RESEARCH SEP 1998, vol. 67, no. 3, September 1998 (1998-09), pages 341-346, XP002698950, ISSN: 0014-4835
- BRUNSCHWEIGER ANDREAS ET AL: "P2 receptors activated by uracil nucleotides--an update.", CURRENT MEDICINAL CHEMISTRY 2006, vol. 13, no. 3, 2006, pages 289-312, XP002698951, ISSN: 0929-8673
- O'BRIEN PAUL D ET AL: "Dry eye: diagnosis and current treatment strategies", CURRENT ALLERGY AND ASTHMA REPORTS, CURRENT SCIENCE, US, vol. 4, no. 4, July 2004 (2004-07), pages 314-319, XP009133136, ISSN: 1529-7322

## Description

### FIELD OF THE INVENTION

The present invention relates to a composition for use in prevention and/or treatment of dry eye syndrome. More specifically, the present invention relates to a composition for use in prevention and treatment of dry eye syndrome, comprising at least one material selected from the group consisting of uridine and triacetyl uridine, as an active ingredient.

### BACKGROUND OF THE INVENTION

Dry eye syndrome is a general term used to describe a disorder of the tear film due to tear deficiency or changes in tear components, which is accompanied by various symptoms of ocular discomfort. According to "Report of the National Eye Institute/Industry Workshop on Clinical Trials in Dry Eyes (1994)", the dry eye syndrome can be defined as an ocular surface disease arising from tear deficiency or excessive tear evaporation that causes damage to the interpalpebral ocular surface and is associated with symptoms of ocular discomfort and ocular irritation. Additionally, the definition of dry eye syndrome has expanded to include any symptomatic external ocular conditions as new types of dry eye syndromes with a neurogenic component (such as dry eyes that occur after LASIK procedures), ocular surface diseases due to an altered tear film composition, and the like have recently occurred (American Journal of Ophthalmology, 140. 507, 2005).

More than 50% of total patients visiting ophthalmic clinics report dry eye syndromes. The elderly, particularly 70 to 80% of post-menopausal women suffer from ocular discomfort due to the dry eye syndrome. In the past, it was considered that the pathological cause of dry eye syndrome was the aqueous layer deficiency resulting from a decrease of tear production by the lacrimal glands. However, recent studies reveal that the primary cause of dry eye syndrome is inflammatory reactions in response to stimuli from the external environment or endogenous inflammatory reactions, thereby presenting problems associated with chronic ocular surface damage arising from disorders of corneal epithelial cells, and decreased interaction between corneal epithelial cells and corneal keratocytes.

The dry eye syndrome may be developed by various etiological causes such as aging, hormonal deficiencies or changes, environmental factors (wind, heat, dust, cigarette smoke, hair dryers, etc.), a chronic low blink rate (VDT syndrome), contact lens wear, LASIK vision correction surgery, medications, and auto-immune diseases (lupus, rheumatoid arthritis and Sjogren's syndrome) (American Journal of Ophthalmology, 137, 337-342, 2004).

In order to treat this syndrome, artificial tears or ointments are usually used to improve ocular lubrication action and to supply moisture on the ocular surface. However, the artificial tear and the like do not exhibit direct anti-inflammatory effects, which are thereby also accompanied by combined treatment of anti-inflammatory agents. As examples of the anti-inflammatory agents, mention may be made of i) tear supplements as an eye drop, such as artificial tear solution, ii) eye drops such as RESTASIS^{™} (cyclosporine ophthalmic emulsion) utilizing inhibitory effects of cytosolic protein cyclophilin-cyclosporine interaction on the activation of T-cells, iii) ophthalmic solution such as Diquafosol, a P2Y2 receptor agonist that stimulates tear and mucin secretion on the ocular surface, thereby treating dry eye, iv) autologous serum eye drops for treating severe dry eye syndrome by improvement of tear stability, and v) steroids.

However, most of these therapeutic agents are treatments for symptomatic relief, not fundamental ones, and therefore they do not present fundamental solutions to the treatment of chronic ocular surface injury arising from decreased interaction between corneal epithelial cells and corneal keratocytes, and dry eye syndrome involving etiological causes including complex and diverse mechanisms and environmental factors. As a result, such current unfavorable circumstances exacerbate the patient's pain. In addition, the chronic abuse of eye drops involves an increase of medical expenses. Further, the steroid drugs elevate intraocular pressure and the prolonged use thereof in chronic diseases and disorders may undesirably cause cataract and glaucoma complications.

To this end, there is an urgent need in the art for the development of an alternative drug which is capable of fundamentally treating and/or preventing dry eye syndrome by assisting the recovery of damaged cells while relieving inflammation without causing adverse side effects.

In this connection, the inventors of the present application have discovered that uridine and derivatives thereof are harmless to humans and facilitate synthesis of hyaluronic acid (HA), thereby being capable of fundamentally treating and/or preventing dry eye syndrome. Based on these findings, we have suggested a composition for treatment and/or prevention of dry eye syndrome, comprising uridine or a derivative thereof as an active ingredient.

The hyaluronic acid (HA) is a naturally occurring biopolymer and is a colorless and transparent linear polysaccharide having a high viscosity and a molecular weight of 50,000 to 13,000,000 Dalton and containing alternating N-acetyl-D-glucosamine and D-glucuronic acid monosaccharide as repeat units.

Viscoelasticity, one of the physical properties of hyaluronic acid, is known to exhibit a cell-protective function by lubrication effects via provision of cushion effects to the eyes and joints, and is also found to take part in wound-healing reaction. Therefore, at the early stage of the inflammatory reaction, the hyaluronic acid increases cell infiltration and cytokine secretion, thus facilitating the wound-healing reaction, and decreases free radicals, thereby participating in anti-oxidative action, consequently playing a role as a mediator of the inflammatory reaction.

Hence, the hyaluronic acid promotes angiogenesis and cell proliferation, thereby assisting in tissue regeneration, reduces unnecessary deposition of collagen, thereby taking part in wound remodeling while leaving small scarring, and also exhibits effects on corneal epithelial regeneration and epidermal regeneration. Therefore, a great deal of research has been recently focused on utilization of the hyaluronic acid as a variety of therapeutic agents. For example, the hyaluronic acid can be used for various applications such as arthritis drugs, dry eye drugs, cosmetic supplements for reduction of wrinkles, anti-adhesion agents used after a surgical operation, cosmetic moisturizers, artificial skin, wound healing, scar-removing agent, cataract surgery assistants, and the like.

In addition, as conventional eye drop solutions containing the hyaluronic acid, there were preparations exerting therapeutic effects via reduction of tear evaporation and lubricating effects and cell regenerating effects. However, the hyaluronic acid observed in epidermal and dermal layers of the human skin has a very short half-life of about 1 day and is regarded as exhibiting high variations in the activity thereof depending upon the difference of molecular weights. Therefore, it was difficult to satisfy the amount of hyaluronic acid necessary for the body only with injection of exogenous hyaluronic acid, while also presenting a skeptical view about the action thereof.

International patent application published under no. WO 98/34593 discloses a sterile preparation adapted for topical administration to the eye, which may comprise phosphates compounds such as uridine 5'-triphosphatc (UTP), adenosine 5'-triphosphate (ATP) and cytidine 5'-triphosphate (CTP) as active ingredients to activate the purinergic receptors in lacrimal tissues.

### SUMMARY OF THE INVENTION

Therefore, the present invention has been made to solve the above problems and other technical problems that have yet to be resolved.

As a result of a variety of extensive and intensive studies and experiments based on the facts as described above, the inventors of the present invention have confirmed that the use of a composition and pharmaceutical preparation, comprising at least one material selected from the group consisting of uridine and triacetyl uridine as an active ingredient, has surprisingly promoted the synthesis of hyaluronic acid (HA), which is harmless to the human body and has various pharmacological effects, and consequently can provide fundamental treatment and/or prevention of dry eye syndrome. The present invention has been completed based on these findings.

### DETAILED DESCRIPTION OF PREFERRED EMBODIMENTS

In accordance with an aspect of the present invention, the above and other objects can be accomplished by the provision of a composition for use in treatment and/or prevention of dry eye syndrome, comprising at least one material selected from the group consisting of uridine and triacetyl uridine, as an active ingredient.

As mentioned above, the composition according to the present invention contains the uridine or triacetyl uridine, such that they induce production of hyaluronic acid by cells or tissues in the human body, thereby continuously promoting the synthesis of endogenous hyaluronic acid leading to continuous supply of hyaluronic acid, serving as a strong moisturizing component, to the eyes. Further, the uridine or triacetyl uridine promote the synthesis of various kinds of glycosaminoglycans (GAGs) and inhibit the production of nitric oxide (NO), in addition to promotion of hyaluronic acid synthesis.

As used herein, the term "glycosaminoglycan" generally refers to mucopolysaccharides such as hyaluronic acid, chondroitin sulfate, heparin, keratin and the like, which are capable of providing lubricating effects, wound-healing effects and moisturizing effects and therefore can be effectively used in the treatment of various diseases arising from deficiency of the mucopolysaccharides including dry eye syndrome. Therefore, the composition containing the uridine and triacetyl uridine according to the present invention can exert excellent therapeutic effects on the treatment or prevention of dry eye syndrome.

The nitric oxide (NO) is an inflammation-related factor in cells and tissues. Expression of inducible nitric oxide synthase (iNOS), a protein involved in the development of inflammation, is increased in the foci of inflammation, which consequently leads to increased production of nitric oxide, thereby exacerbating inflammation. Hence, reduction of nitric oxide production is recognized as an effective therapeutic method of inflammation. Meanwhile, the dry eye syndrome is also accompanied by wound-induced inflammation. Therefore, the uridine and triacetyl uridine according to the present invention can decrease production of nitric oxide or can effectively scavenge nitric oxide, and as a result, can exert excellent effects on the inflammation treatment of dry eye syndrome.

Consequently, the composition of the present invention provides treatment and/or prevention of dry eye syndrome, mediation of inflammation and recovery of damaged corneal epithelial cells and corneal keratocytes, thus being capable of treating epithelial cell disorders, and therefore can also be helpful for treatment of corneal epithelial disorders caused by secondary factors.

Further, conventional direct injection of exogenous hyaluronic acid can merely obtain topical (local) effects, thus providing limited therapeutic effects. The composition containing the uridine and triacetyl uridine according to the present invention can solve these problems suffered by conventional arts, and enables administration thereof via various routes including oral administration, thereby achieving rapid therapeutic effects and safe treatment.

The uridine is one of four ribonucleosides necessary for constitution of RNA, is widely distributed in nature, and has a molecular weight of about 244.20 and a molecular formula of C₉H₁₂N₂O₆. Pharmacological activities of uridine known hitherto are as follows. For example, it is known that, upon *in vivo* administration of uridine, uridine is converted into uridine 5'-phospate by catalysis of uridine kinase, thereby being useful for enzyme deficiency or pyrimidine nucleotide deficiency and deficiency of nucleic acid synthesis, and thus it may be used as a drug relieving orotic acidonuria (MARTINDALE, 1989, 1627). In addition, it is also known that uridine can be used as a therapeutic agent for collagen arthritis (Korean Patent Publication No. 1994-0008033 B1, assigned to Green Cross Holdings Corp., Korea), or uridine in the form of a composite preparation with other pharmacologically active substances can also be used in the treatment of vascular cerebral diseases, hepatic diseases, peripheral neuropathy and tissue hypoxia. However, none of the above-mentioned publications or patents disclosed or suggested prevention and treatment of dry eye syndrome via promotion of the hyaluronic acid synthesis, as demonstrated in the present invention.

The present inventors have newly found that the uridine or triacetyl uridine promote the synthesis of hyaluronic acid as well as the synthesis of glycosaminoglycans and inhibit production of nitric oxide (NO), and are thereby capable of remarkably treating dry eye syndrome. These findings and facts can also be demonstrated through the following examples.

Specifically, the present inventors have measured the activity of the uridine and triacetyl uridine on the promotion of hyaluronic acid and glycosaminoglycan production and the inhibition of nitric oxide (NO) production in corneal keratocytes and epithelial cells, and as a result, have confirmed that the uridine and triacetyl uridine exhibit excellent effects on the treatment of dry eye syndrome with the restoration of epithelial cells. On the other hand, it was confirmed that the uridine or triacetyl uridine exhibit an excellent therapeutic activity as both an eye drop and oral preparation, and a facilitation degree of hyaluronic acid and glycosaminoglycan production and an inhibition degree of nitric oxide (NO) production correlate with concentrations of the uridine or triacetyl uridine.

As a result, the composition for prevention and treatment of dry eye syndrome containing the uridine and/or triacetyl uridine as the active ingredient can prevent and treat dry eye syndrome through the recovery of epithelial cells, and thus it is considered that they can be developed as various therapeutic agents for numerous diseases associated with the dry eye syndromes. The composition for prevention and treatment of dry eye syndrome in accordance with the present invention comprises the above uridine and/or triacetyl uridine as the active ingredient, and may be formulated into prophylactic and therapeutic agent of dry eye syndrome, in conjunction with addition of a pharmaceutically acceptable carrier, if necessary.

. The content of the active ingredient may be in the range of preferably, 0.1 to 20% by weight, more preferably 1 to 10% by weight, based on the total weight of the composition.

Preferably, the composition may be a pharmaceutical composition containing a pharmaceutically acceptable carrier or vehicle.

A suitable dose of the pharmaceutical composition of the present invention may vary depending upon various factors such as formulation method, administration mode, age, weight and sex of patients, pathological conditions, diet, administration time, administration route, excretion rate and sensitivity to response.

The uridine or triacetyl uridine can be administered via oral or parenteral routes upon clinical administration and can be used in various forms of general pharmaceutical formulations. That is, the composition of the present invention may be administered in the form of various oral and parenteral formulations, upon practical clinical administration. When formulating of the composition into desired dosage forms, the formulations are prepared using conventional filling agents, extenders, binding agents, wetting agents, disintegrating agents, and diluents or vehicles such as surfactants. Solid formulations for oral administration include, for example, tablets, pills, powders, granules and capsules, and are prepared by mixing the uridine or triacetyl uridine with one or more vehicles, such as starch, calcium carbonate, sucrose, lactose and gelatin. Lubricating agents such as magnesium stearate and talc may also be used, in addition to simple vehicles. As liquid formulations for oral administration, mention may be made of suspensions, solutions for internal use, emulsions and syrups. In addition to generally used simple diluents such as water and liquid paraffin, the above-mentioned formulations can contain various vehicles, for example wetting agents, sweetening agents, aromatics and preservatives. Formulations for parenteral administration include sterile aqueous solutions, non-aqueous solvents, suspensions, emulsions, lyophilized formulations and suppositories. As non-aqueous solvents and suspensions, there may be used propylene glycol, polyethylene glycol, vegetable oil such as olive oil, injectable ester such as ethylolate, etc. As base materials for suppositories, Witepsol, macrogol, Tween 61, cacao butter, laurin butter, glycerol and gelatin may be used.

Dosage units may contain one-, two-, three- or four-fold amount of an individual dose, or 1/2, 1/3 or 1/4-fold amount of an individual dose. Preferably, the individual dose contains an amount of the active drug that is administered one time, and typically corresponds to the total amount administered for one day, or 1/2, 1/3 or 1/4 fold-amount thereof. Although effective doses of the uridine or triacetyl uridine are concentration-dependent, they are preferably in the range of 0.1 to 1,000 mg/kg, more preferably 0.4 to 200 mg/kg and may be administered 1 to 6 times a day.

Preferably, the composition may be formulated into a composition containing one or more cosmetically or nutritionally acceptable carriers or vehicles. That is, the composition may be prepared into a composition for prevention and treatment of dry eye syndrome in the form of a cosmetic or cosmetic additive, a beverage or beverage additive, or a food or food additive.

In accordance with another aspect of the present invention, there is provided a functional health food for preventing and/or treating dry eye syndrome, containing uridine or triacetyl uridine as an active ingredient. The term "a functional health food" used throughout the specification of the present invention refers to a food in which uridine or triacetyl uridine are added to general foods to improve functions thereof. The uridine or triacetyl uridine may be added to general foods or may be prepared in the form of capsules, powders, suspensions and the like. Intake of such a functional health food containing uridine or triacetyl uridine provides beneficial effects for health, and exhibits advantages in that there are no adverse side effects which may occur upon prolonged use of drugs because food material is used as the raw material, unlike conventional drugs.

If it is desired to use uridine or triacetyl uridine of the present invention as the food additive, the uridine or triacetyl uridine can be added alone, or can be used in conjunction with other foods or food ingredients, or may be used appropriately according to other conventional methods. Mixed amounts of active ingredients may be suitably determined depending upon the purpose of use (prophylactic, health or therapeutic treatment). Generally, upon production of foods or beverages with which the uridine or triacetyl uridine are mixed, these materials may be added preferably in an amount of 0.1 to 20% by weight, based on the total weight of raw materials. However, when prolonged intake is intended for the purpose of health and hygiene or for health control, the above-mentioned amount of the uridine or triacetyl uridine may be adjusted below the above-specified range. In addition, the health food of the present invention preferably contains uridine or triacetyl uridine in amounts falling within the determined toxicity range, when it is employed as the pharmaceutical composition as mentioned above.

There is no particular limit to kinds of the above-mentioned foods. As examples of foods to which the uridine or triacetyl uridine can be added, mention may be made of meats, sausages, bread, chocolate, candies, snack, confectionary, pizza, Ramen, other noodles, gum, skimmed milk, dried foods, raw foods, dairy products including lactic acid bacteria-fermented milk and ice cream, various soups, beverages, teas, drinks, alcoholic beverages and multi-vitamin preparations. Specifically, as examples of health foods containing the uridine or triacetyl uridine, mention may be made of health foods and special favorite products such as squeezed liquid, tea, jelly and juice made of the uridine or triacetyl uridine as main ingredients. In addition, mention may be made of folk medicines for edema, nephritis and urethritis as target diseases or disorders to be treated.

When it is desired to use the uridine or triacetyl uridine of the present invention as cosmetic raw materials, they can be added by themselves or can be used in conjunction with other cosmetic ingredients, or may be used appropriately according to other conventional methods. Mixed amounts of active ingredients may be suitably determined depending upon the purpose of use thereof. Generally, in producing cosmetics using the uridine or triacetyl uridine, these materials may be added preferably in the amount of 0.1 to 20% by weight, based on the total weight of raw materials. Cosmetics include, but are not limited to, aftershaves, lotions, creams, packs and color cosmetics.

In accordance with yet another aspect of the present invention, there is provided a preparation for prevention and/or treatment of dry eye syndrome, comprising the above-mentioned composition as an active ingredient, and one or more pharmaceutically acceptable carriers or vehicles.

Therefore, the above-mentioned preparation may contain pharmaceutically acceptable carrier, diluent, vehicle or any combination thereof, if necessary. These components facilitate administration of the active ingredient into the organism.

The carrier is defined as a compound that facilitates addition of a compound or substance of interest into target cells or tissues. Although there is no particular limit to kinds of carriers, the carrier may be preferably one conventionally used in the art depending upon desired formulations, for example at least one selected from the group consisting of solid carriers such as starch, lactose, mannitol, carboxymethylcellulose, corn starch and inorganic salts; liquid carriers such as distilled water, physiological saline, aqueous glucose solutions, alcohols such as ethanol, propylene glycol, and polyethylene glycol; and oily carriers such as various animal and vegetable oils, white Vaseline, paraffin and wax.

Examples of vehicles may include fillers such as lactose, sucrose, mannitol and sorbitol, corn starch, wheat starch, rice starch, potato starch, gelatin, tragacanth gum, methyl cellulose, hydroxypropylmethylcellulose, sodium carboxymethylcellulose, and/or cellulosic materials such as polyvinylpyrrolidone (PVP).

Preparation of the composition into the pharmaceutical formulation may be carried out by conventional methods known in the art. Preferably, the pharmaceutical formulation may be oral, external, transdermal or transmucosal preparation which is pharmaceutically acceptable. For example, the pharmaceutical formulation may be a powdered drug, a granule, a solution, a pill, a troche, a suspension, an emulsion, a syrup, a tablet, a powder, a soft or hard capsule, a suspension, an injectable preparation or an emulsified liquid, or a single- or multi-dose preparation for parenteral administration. More preferably, the pharmaceutical formulation may be an oral preparation or an eye drop. Particularly, the oral preparation according to the present invention can exert superior effects almost comparable to use of the eye drop. Such a fact can also be confirmed through the following examples.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a graph illustrating a degree of hyaluronic acid synthesis in corneal keratocytes, depending upon different concentrations of uridine;
FIG. 2 is a graph illustrating a degree of glycosaminoglycan synthesis in corneal keratocytes, depending upon different concentrations of uridine;
FIG. 3 is a graph illustrating effects of uridine on production of nitric oxide (NO) in corneal keratocytes, depending upon different concentrations of uridine;
FIG. 4 is a graph illustrating effects of uridine on proliferation of corneal keratocytes;
FIG. 5 is a graph illustrating a degree of glycosaminoglycan synthesis in ocular epithelial cells, depending upon different concentrations of uridine;
FIG. 6 is a graph illustrating effects of uridine on production of nitric oxide (NO) in ocular epithelial cells;
FIG. 7 is a graph comparing alleviation in dry eye symptom between a treatment group and a control group, after treatment of dry eye-induced rabbits with a uridine eye drop;
FIG. 8 is a graph showing results of a tear-secretion test (Schirmer test), after oral administration of uridine into dry eye patients; and
FIG. 9 is a graph showing results of corneal staining, after oral administration of uridine into dry eye patients.

### EXAMPLES

Now, the present invention will be described in more detail with reference to the following Examples. These examples are provided only for illustrating the present invention and should not be construed as limiting the scope and spirit of the present invention.

### Example 1 : Isolation of corneal keratocytes and corneal epithelial cells

Ocular blood vessels were removed from corneal tissues which were cleared. The then cleared tissues were washed with 5% Antibiotics-PBS, placed in 3 mL of 1X Trypsin (0.05%)/EDTA (0.01%) and then subjected to shaking incubation at 37°C for 80 min. The resulting suspension was pooled and washed with a SHEM media (10% FBS in DMEM/F12(1:1) + 0.5% PS + 4 mM L-glutamine + 10 ng/mL EGF + 5 µg/mL insulin + 30 ng/mL cholera toxin + 0.18 mM adenine + 2 nM 3,3',5-triiodo-L-thyronine sodium salt + 0.4 µg/mL hydrocortisone), and centrifuged at 1,200 rpm for 5 min. At this time, 1.2 U/mL of Dispase II was added to the remaining tissues after use thereof as the epithelium and the isolated cells were incubated at 37°C for 2 hours. Next, 1.2 U/mL of Dispase II was removed, and 200 U/mL of type I collagenase (5 mL) was treated on cells which were then subjected to shaking incubation at 37°C. The cell suspension was pooled, washed with culture media (10% FBS in DMEM/F12(1:1) + 1% PS), centrifuged at 1,200 rpm for 5 min and plated on a dish.

### Example 2: Effects of uridine on production of hyaluronic acid in corneal keratocytes

Corneal keratocytes were stimulated for 48 hours by treatment of the cells with uridine (0 µM to 10 µM). 100 µℓ of the cell culture was collected, and reacted with 100 µℓ of biotinylated hyaluronic acid binding protein (b-HABP, 50 ng/mL) at room temperature for 1 hour.

On the day prior to experiments, 20 µg/mL of HA and a solution of 20 mM Na₂CO₃ were added to a 96-well plate which was then stored in a refrigerator for 24 hours, and washed with a standard assay buffer (SAB) three times. 1% BSA was added to the 96-well plate which was then reacted at room temperature for 90 min and washed with SAB three times to thereby prepare an HA-coated plate.

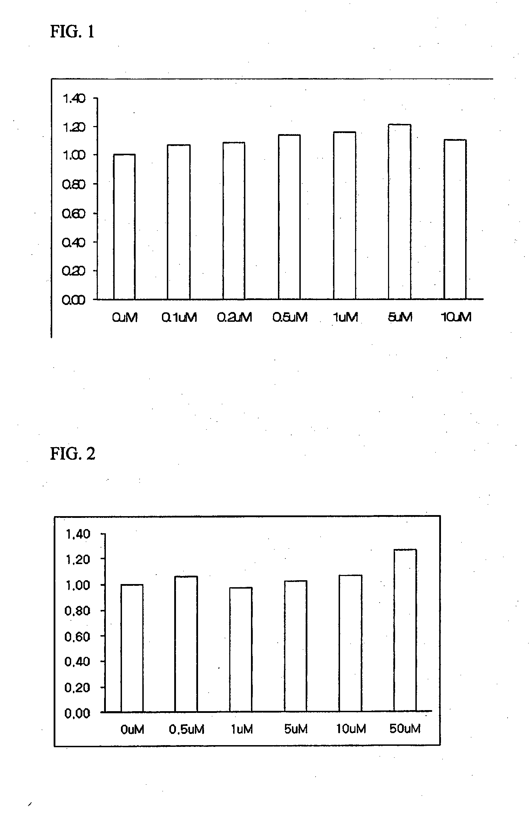

The above-prepared cell culture was added to the HA-coated plate, and reacted with stirring at room temperature for 1 hour. Thereafter, the cell culture thus reacted was washed with SAB three times, followed by addition of Extra-avidin alkaline phosphatase (1:80000), reaction at room temperature for 30 min, and washing with SAB three times. 1 mg/mL of a disodium p-nitrophenyl solution in 100 mM NaCl and 5 mM MgCl was added to the cell culture which was then reacted for 20 to 60 min. Thereafter, 3N NaOH was added to terminate the reaction, and an amount of hyaluronic acid was measured at 405 nm. The results thus obtained are given in FIG. 1.

As shown in FIG. 1, it was confirmed that treatment of corneal keratocytes with uridine promoted production of hyaluronic acid at a uridine concentration up to 5 µM, in a concentration-dependent manner.

### Example 3: Effects of uridine on production of glycosaminoglycan in corneal keratocytes

Corneal keratocytes were stimulated for 48 hours by treatment of the cells with uridine (0 µM to 50 µM). The cell culture was collected, and an amount of glycosaminoglycan was measured at 550 nm using a Biocolor assay kit. The results thus obtained are given in FIG. 2. As shown in FIG. 2, it was confirmed that treatment of corneal keratocytes with uridine promoted production of glycosaminoglycan from a uridine concentration of 1 µM, in a concentration-dependent manner.

### Example 4: Effects of uridine on production of nitric oxide (NO) in corneal keratocytes

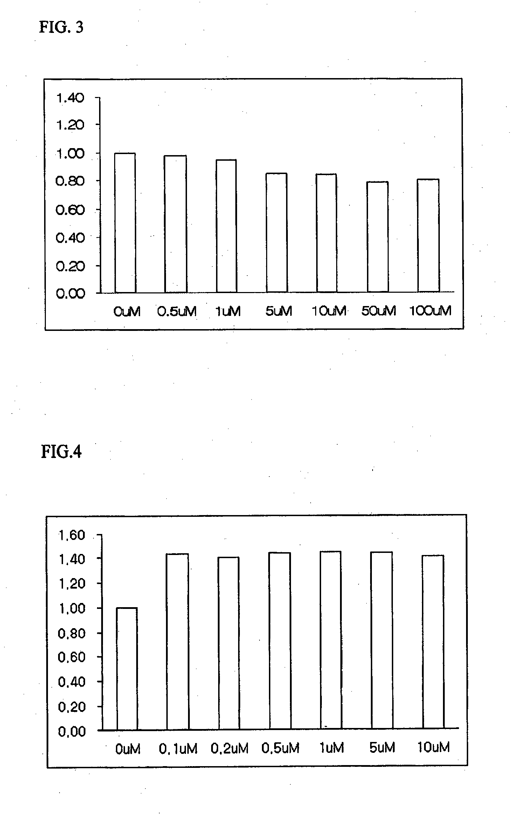

Corneal keratocytes were stimulated with 100 µM H₂O₂ followed by stimulation with uridine (0 µM to 100 µM) for 48 hours. 100 µℓ of Griess reagent (4 µg/mL) was added to the cell suspension, and the resulting mixture was reacted with stirring at room temperature for 15 min. An amount of nitric oxide (NO) was measured at 540 nm. The results thus obtained are given in FIG. 3. As shown in FIG. 3, it was confirmed that treatment of corneal keratocytes with uridine inhibited production of nitric oxide (NO) at a uridine concentration of up to 50 µM, in a concentration-dependent manner.

### Example 5: Effects of uridine on proliferation of corneal keratocytes,

Corneal keratocytes were incubated in a 96-well plate and stimulated with uridine (0 µM to 10 µM) for 48 hours. Cell count was carried out at 450 nm using a Cell counting kit-8 (CCK-8). The results thus obtained are given in FIG. 4. As shown in FIG. 4, it was revealed that treatment of corneal keratocytes with uridine promoted proliferation of cells from a uridine concentration of 0.1 µM or higher.

### Example 6: Effects of uridine on production of glycosaminoglycan in corneal epithelial cells

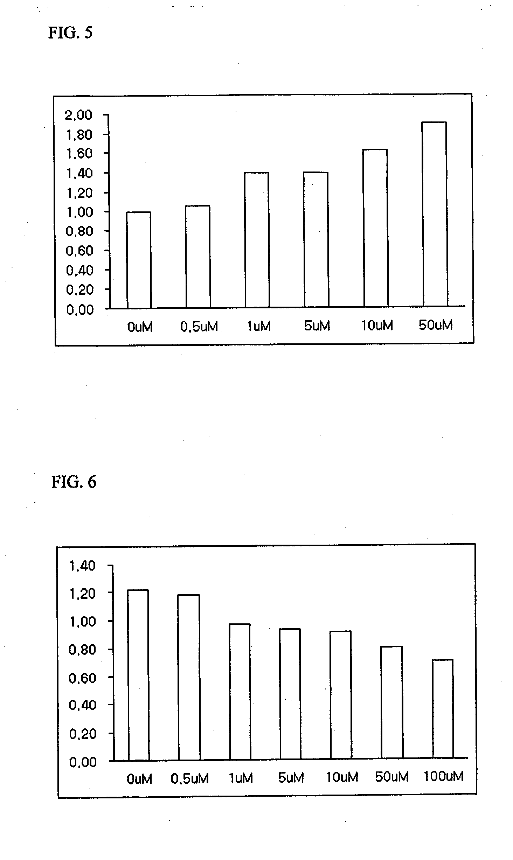

Ocular epithelial cells were stimulated for 48 hours by treatment with uridine (0 µM to 50 µM). The cell culture was collected, and an amount of glycosaminoglycan was measured at 550 nm using a Biocolor assay kit. The results thus obtained are given in FIG. 5. As shown in FIG. 5, it was confirmed that treatment of epithelial cells with uridine exhibited promoted production of glycosaminoglycan, in a concentration-dependent manner.

### Example 7: Effects of uridine on production of nitric oxide (NO) in corneal epithelial cells

Ocular epithelial cells were stimulated with 100 µM H₂O₂ followed by stimulation with uridine (0 µM to 100 µM) for 48 hours. 100 µℓ of Griess reagent (4 µg/mL) was added to the cell suspension, and the resulting mixture was reacted with stirring at room temperature for 15 min. An amount of nitric oxide (NO) was measured at 540 nm. The results thus obtained are given in FIG. 6. As shown in FIG. 6, it was confirmed that treatment of ocular epithelial cells with uridine inhibited production of nitric oxide (NO), in a concentration-dependent manner.

### Example 8: In vivo animal experiments

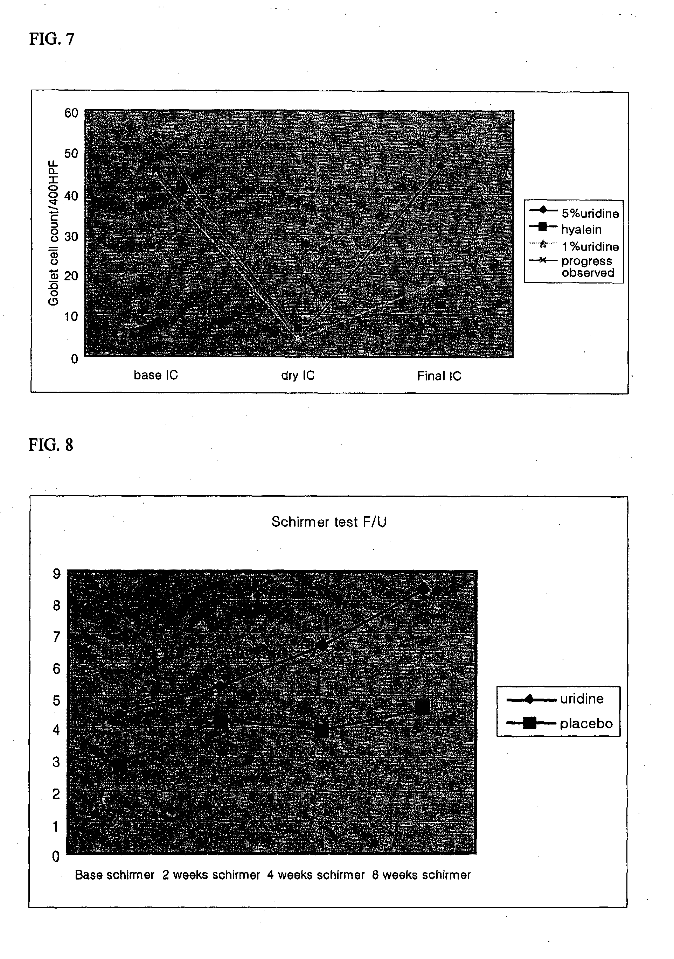

Dry eye syndrome was induced by causing ocular inflammation via injection of concanavalin A into the lacrimal gland of rabbits (Journal of Ocular Pharmacology & Therapeutics 21(2), 139-148, 2005). Uridine was dissolved to a concentration of 5% and 10% in a phosphate-buffered saline solution, respectively, and the resulting solutions were used as eye drops. A commercially available 0.1% hyaluronic acid preparation was used as a positive control group, and a phosphate-buffered saline solution was used as a control group. 1% and 5% uridine preparations, the hyaluronic acid preparation and the phosphate-buffered saline solution were respectively dropped into eyes of the dry eye-induced rabbits, and the impression cytology of ocular cells was performed to confirm alleviation of dry eye syndrome. The results thus obtained are given in FIG. 7.

As shown in FIG. 7, it was confirmed that the eye drops using uridine exhibited significant restoration of epithelial cells in the impression cytology of ocular cells, as compared to the positive control group and the control group.

### Example 9: Clinical tests

After obtaining the written informed consent forms of trial subjects from dry eye patients who do not have other ocular diseases except for dry eye, exhibit no further amelioration of conditions even with at least two-month administration of conventional drugs, secrete tears of less than 5 mm in a tear-secretion test (Schirmer test), and are in need of surgical treatment including nasolacrimal duct obstruction, oral preparations containing uridine (100 mg/day) and oral placebo preparations containing no uridine were orally administered to patients three times a day for 3 months, by the double blind method. At initial point of treatment and two weeks, one month, two months and three months after initiation of treatment, changes in corneal symptoms and conditions and the presence/absence of adverse side effects were objectively evaluated by slit-lamp microscopic examination, measurement of intraocular pressure, ophthalmoscopic examination, Schirmer test, impression cytology and analysis photography of anterior eye segment. In addition, subjective questionnaires of individual patients were prepared, and changes in subjective symptoms of patients and the presence/absence of adverse side effects were observed.

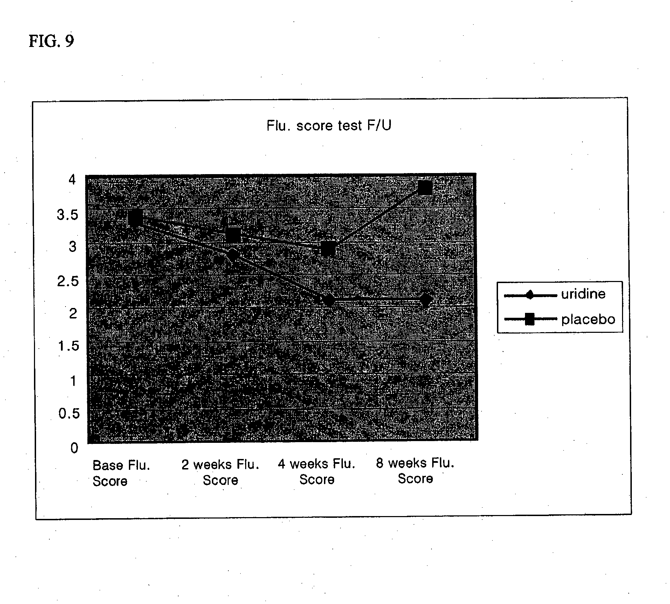

FIG. 8 is a graph showing results of a tear-secretion test (Schirmer test), and FIG. 9 is a graph showing results of corneal staining. Referring to FIGS. 8 and 9, the uridine-administered groups exhibited a significant difference in Schirmer test and corneal staining, thus confirming that application of uridine as both the eye drop and the oral preparation is therapeutically effective for the treatment of dry eye syndrome.

### INDUSTRIAL APPLICABILITY

As apparent from the foregoing, use of compositions and pharmaceutical preparations of the present invention, which comprise at least one material selected from the group consisting of uridine and triacetyl uridine as an active ingredients, facilitates synthesis of hyaluronic acid in the human cell and body, and therefore can prevent and treat the dry eye syndrome.

Although the preferred embodiments of the present invention have been disclosed for illustrative purposes, those skilled in the art will appreciate that various modifications, additions and substitutions are possible, without departing from the scope of the invention as disclosed in the accompanying claims.

## Claims

1. A composition for use in prevention and/or treatment of dry eye syndrome, **characterized in that** the active ingredient consist of uridine or triacetyl uridine or combination thereof as an active ingredient.

2. The composition for use according to claim 1, wherein the content of the active ingredient is in the range of 0.1 to 20% by weight, based on the total weight of the composition.

3. The composition for use according to any one of claims 1 or 2, wherein the composition is a cosmetic composition containing a cosmetically acceptable carrier or vehicle.

4. The composition for use according to any one of claims 1 or 2, wherein the composition is a food or beverage composition containing a nutritionally acceptable carrier or vehicle.

5. The composition for use according to any one of claims 1 to 3, wherein the composition is a pharmaceutical composition containing a pharmaceutically acceptable carrier or vehicle.

6. A therapeutic preparation for use in prevention and/or treatment of dry eye syndrome, comprising the composition of claim 1 as an active ingredient, and one or more pharmaceutically acceptable carriers or vehicles.

7. The therapeutic preparation for use according to claim 6, wherein the carrier is at least one selected from the group consisting of solid carriers such as starch, lactose, mannitol, carboxymethylcellulose, corn starch and an inorganic salt; liquid carriers such as distilled water, physiological saline, an aqueous glucose solution, an alcohol such as ethanol, propylene glycol, and polyethylene glycol; and oily carriers such as various animal and vegetable oils, white Vaseline, paraffin and wax.

8. The therapeutic preparation for use according to claim 6, wherein the content of the active ingredient is in the range of 0.1 to 20% by weight.

9. The therapeutic preparation for use according to claim 6, wherein the preparation is a powdered drug, a granule, a solution, a pill, a troche, a suspension, an emulsion, a syrup, a tablet, a powder, a soft or hard capsule, a suspension, an injectable preparation, an emulsified liquid, or a single- or multi-dose preparation for parenteral administration.

10. The therapeutic preparation for use according to claim 6, wherein the preparation is an oral preparation or an eye drop.

11. The therapeutic preparation for use according to claim 9, wherein the preparation contains a pharmaceutically acceptable vehicle, such that it can be formulated in the form of an edible beverage, food or cosmetic.

## Patentansprüche

1. Eine Zusammensetzung zur Verwendung in der Prävention und/oder Behandlung eines Trockenes-Auge-Syndroms, **gekennzeichnet dadurch, dass** der Wirkstoff aus Uridin oder Triacetyluridin oder einer Kombination davon als einem Wirkstoff besteht.

2. Die Zusammensetzung zur Verwendung nach Anspruch 1, wobei der Gehalt des Wirkstoffs im Bereich von 0,1 bis 20 Gew%, bezogen auf das Gesamtgewicht der Zusammensetzung, liegt.

3. Die Zusammensetzung zur Verwendung nach einem der Ansprüche 1 oder 2, wobei die Zusammensetzung eine kosmetische Zusammensetzung ist, welche einen kosmetisch akzeptablen Träger oder Bindemittel enthält.

4. Die Zusammensetzung zur Verwendung nach einem der Ansprüche 1 oder 2, wobei die Zusammensetzung eine Nahrungsmittel- oder Getränkezusammensetzung ist, die einen ernährungsphysiologisch akzeptablen Träger oder Bindemittel enthält.

5. Die Zusammensetzung zur Verwendung nach einem der Ansprüche 1 bis 3, wobei die Zusammensetzung eine pharmazeutische Zusammensetzung ist, die einen pharmazeutisch akzeptablen Träger oder Bindemittel enthält.

6. Ein therapeutisches Präparat zur Verwendung in der Prävention und/oder Behandlung eines Trockenes-Auge-Syndroms, umfassend die Zusammensetzung nach Anspruch 1 als einen Wirkstoff und einen oder mehrere pharmazeutisch akzeptable Träger oder Bindemittel.

7. Das therapeutische Präparat zur Verwendung nach Anspruch 6, wobei der Träger mindestens einer, ausgewählt aus der Gruppe, bestehend aus festen Trägern wie Stärke, Laktose, Mannitol, Carboxymethylcellulose, Maisstärke und einem anorganischen Salz; flüssigen Trägern, wie destilliertes Wasser, physiologische Kochsalzlösung, eine wässrige Glukose-Lösung, ein Alkohol wie Ethanol, Propylenglykol und Polyethylenglykol; und öligen Trägern wie verschiedenen tierischen und pflanzlichen Ölen, weißem Vaselin, Paraffin und Wachs, ist.

8. Das therapeutische Präparat zur Verwendung nach Anspruch 6, wobei der Gehalt des Wirkstoffs im Bereich von 0,1 bis 20 Gew% liegt.

9. Das therapeutische Präparat zur Verwendung nach Anspruch 6, wobei das Präparat ein pulverförmiges Arzneimittel, ein Granulat, eine Lösung, eine Tablette, eine Pastille, eine Suspension, eine Emulsion, ein Sirup, eine Tablette, ein Pulver, eine weiche oder harte Kapsel, eine Suspension, ein injizierbares Präparat, eine emulgierte Flüssigkeit, oder ein Ein- oder Mehrfach-Dosis-Präparat für die parenterale Verabreichung ist.

10. Das therapeutische Präparat zur Verwendung nach Anspruch 6, bei der das Präparat ein orales Präparat oder Augentropfen ist.

11. Das therapeutische Präparat zur Verwendung nach Anspruch 9, wobei das Präparat ein pharmazeutisch akzeptables Bindemittel beinhaltet, so dass es in der Form eines verzehrbaren Getränkes, Lebensmittels oder Kosmetikums fomuliert werden kann.

## Revendications

1. Une composition à utiliser dans la prévention et/ou le traitement du syndrome des yeux secs, caractérisée en ce le principe actif consiste en de l'uridine ou de la triacyl-uridine ou une combinaison des deux en tant que principe actif.

2. La composition à utiliser selon la revendication 1, dans laquelle la teneur en principe actif est comprise dans la plage allant de 0,1 à 20% en masse, par rapport à la masse totale de la composition.

3. La composition à utiliser selon l'une ou l'autre des revendications 1 et 2, dans laquelle la composition est une composition cosmétique contenant un vecteur ou un support cosmétiquement acceptable.

4. La composition à utiliser selon l'une ou l'autre des revendications 1 et 2, dans laquelle la composition est une composition alimentaire ou une boisson contenant un vecteur ou un support nutritionnellement acceptable.

5. La composition à utiliser selon l'une ou l'autre des revendications 1 à 3, dans laquelle la composition est une composition pharmaceutique contenant un vecteur ou un support phannaceutiquement acceptable.

6. Une préparation thérapeutique à utiliser dans la prévention et/ou le traitement du syndrome des yeux secs, comprenant la composition de la revendication 1 comme principe actif et un ou plusieurs vecteurs ou supports pharmaceutiquement acceptables.

7. La préparation thérapeutique à utiliser selon la revendication 6, dans laquelle le vecteur est au moins un élément sélectionné parmi le groupe constitué par les vecteurs solides tels que l'amidon, le lactose, le mannitol, la carboxy-méthyl-cellulose, l'amidon de maïs et un sel inorganique ; les vecteurs liquides tels que l'eau distillée, un sérum physiologique, une solution aqueuse de glucose, un alcool tel que l'éthanol, le propylène glycol, et le polyéthylène glycol ; et les vecteurs huileux tels que diverses huiles animales et végétales, la vaseline blanche, la paraffine et la cire.

8. La préparation thérapeutique à utiliser selon la revendication 6, dans laquelle la teneur en principe actif est dans la plage allant de 0,1 à 20% en masse.

9. La préparation thérapeutique à utiliser selon la revendication 6, dans laquelle la préparation est un médicament en poudre, un granulé, une solution, une pilule, une pastille, une suspension, une émulsion, un sirop, un comprimé, une poudre, une gélule ou capsule, une suspension, une préparation injectable, un liquide émulsifé ou une préparation pour administration parentérale mono- ou multi-dose.

10. La préparation thérapeutique à utiliser selon la revendication 6, dans laquelle la préparation est une préparation orale ou des gouttes oculaires.

11. La préparation thérapeutique à utiliser selon la revendication 9, dans laquelle la préparation contient un vecteur pharmaceutiquement acceptable, de telle manière qu'elle peut être formulée sous la forme d'une boisson consommable, de nourriture ou de cosmétique.
